# EUROPÄISCHE PATENTANMELDUNG

(11) **EP 1 156 336 A1**
(43) Veröffentlichungstag der Anmeldung: **21.11.2001**
(21) Anmeldenummer: 00890155.5
(22) Anmeldetag: 16.05.2000
(51) Int. Cl.: G01N 35/00, A61B 5/00, G01N 33/487, G06F 19/00

(54) **Analysensystem zur Analyse medizinischer Proben**

(71) Anmelder: AVL Medical Instruments AG, 8207 Schaffhausen (CH)
(72) Erfinder: Berger, Hans, Dr., 8020 Graz (AT); Kanter, Ulrich, Dipl.-Ing., 8141 Unterpremstätten (AT); Karpf, Hellfried, Dr., 8043 Graz (AT); Ziegler, Werner, Dipl.-Ing., 8043 Graz (AT)
(74) Vertreter: Babeluk, Michael, Dipl.-Ing. Mag.

(57) **Zusammenfassung**

Ein flexibles, erweiterungsfähiges Analysensystem zur Analyse medizinischer Proben, vorzugsweise zur Analyse von Körperflüssigkeiten weist ein oder mehrere, autonome Einzelanalysatoren (3) zur Messung jeweils eines Probenparameters oder einer Parametergruppe und eine rechnergestützte Zentraleinheit (1) mit einer Ein- und Ausgabeeinheit (5, 6, 7, 8) auf. Die Einzelanalysatoren (3) sind in einer ersten Position, der Ladeposition, mit der Zentraleinheit (1) gekoppelt, aus der Ladeposition entnehmbar und in einer zweiten, vorzugsweise patientenseitigen Messposition einsetzbar. Die in der Ladeposition an die Zentraleinheit (1) gekoppelten Einzelanalysatoren (3) bilden einen Mehrkomponentenanalysator.

## Beschreibung

Die Erfindung betrifft ein Analysensystem zur Analyse medizinischer Proben, vorzugsweise zur Analyse von Körperflüssigkeiten.

Es sind bereits eine Vielzahl von elektrochemischen bzw. elektrooptische Analysatoren auf dem Gebiet der Labor- bzw. Medizintechnik im Einsatz, welche unterschiedlichsten Anforderungen genügen müssen. Die Auswahl erstreckt sich von einfachen, im wesentlichen manuell bedienbaren Geräten bis hin zu komplexen, hoch automatisierten Analysatoren und Analysensystemen. Meist sind in den Labors unterschiedlichste Analysatoren unterschiedlicher Hersteller nebeneinander anzutreffen, welche unterschiedlichste Bedienungsschritte für die Probenvorbereitung, den Messetrieb, die Wartung und die Dokumentation der gewonnenen Messdaten erfordern. Vielfach werden in den Labors für die in großer Zahl anfallenden Proben hoch automatisierte Analysatoren mit raschem Probendurchsatz benötigt, welche meist nur eine geringe Menüauswahl zulassen. Für andere Probenparameter, welche seltener gemessen werden, müssen oft zusätzliche Analysatoren angeschafft werden, welche neben den zusätzlichen Kosten zusätzliches Bedienungspersonal, zusätzlichen Laborplatz, eine separate Probenvorbereitung und eine aufwendige Zuordnung und Dokumentation der Messdaten erfordern.

Es wurden deshalb Analysensysteme verwirklicht, bei welchen zwei Analysatoren mit unterschiedlichem Probendurchsatz und unterschiedlichen Analysenmöglichkeiten zu einem modularen Analysensystem zusammengefasst werden, wobei die Vorteile der Einzelgeräte kombiniert werden. Ein derartiges System wird beispielsweise in der US 4,965,049 A beschrieben. Beide Einzelanalysatoren des Analysensystem verfügen über jeweils ein Probenkarussell, entsprechende Einrichtungen zur Probenanalyse sowie verschwenkbare Probentnahmeelemente, welche die Probe aus dem Probenkarussell in die Analysiereinrichtungen transportieren. Nach Abnahme der Seitenelemente benachbarter Gehäuseteile beider Einzelanalysatoren können diese nach genauer Justierung mittels Distanzelementen zu einem Analysensystem verbunden werden, so dass das verschwenkbare Probentnahmeelement des ersten Analysators Zugriff zu den Proben im Probenkarussell des zweiten modularen Analysators hat. Da alle Proben des Analysensystems über das Probenkarussell des zweiten Analysators eingegeben werden, kann nach der Kombination beider Analysatoren das Probenkarussell des ersten Analysators entfernt werden.

Zur Synchronisation des verschwenkbaren Probentnahmeelementes des ersten Analysators mit dem Probenkarussell des zweiten Analysators sowie zum Austausch der Messdaten zwischen den beiden Analysatoren sind beide Analysatoren über einen Datenbus verbunden. Weiters verfügen beide Analysatoren über ein gemeinsames System für die benötigte Waschlösung, wobei durch die benachbarten Seitenwände entsprechende Schlauchleitungen durchgeführt sind.

Nachteilig bei diesem System ist die Tatsache, dass mit einfachen Mitteln kaum Erweiterungen durch zusätzliche Einzelanalysatoren möglich sind, sowie dass nach dem Zusammenfügen der beiden Einzelanalysatoren zu einem Analysensystem die Verwendung der beiden Analysatoren als separate, unabhängig an verschiedenen Orten einsetzbare Analysatoren nur nach zeitaufwendigen Demontageschritten möglich ist.

Weiters werden im Critical-Care-Bereich seit längerem benutzerfreundliche, automatisierte Analysatoren eingesetzt, welche eine Vielzahl unterschiedlicher Parameter bzw. Parametergruppen einer medizinischen Probe bestimmen können. Ein Beispiel dafür ist der modular aufgebaute Analysator AVL-OMNI (AVL Medical Instruments AG, Schaffhausen, CH), welcher über eine Probeneingabe und mehrere für die jeweilige Analyse beliebig anwählbare Messmodule aufweist. Unter anderem können in den Analysator Module zur Messung der Blutgase (pH *P*CO₂, *P*O₂), zur Messung der Elektrolyten (Na⁺, K⁺, Cl⁻, Ca⁺⁺), zur Bestimmung von Hämoglobin und für die CO-Oximetrie eingesetzt werden. Das komplexe Gerät, welches mit einem benutzerfreundlichen Touch-Screen, einem Thermodrucker und einer hochwertigen Auswerteelektronik samt Speicher für die Patientendaten ausgestattet ist, eignet sich hervorragend für den medizinischen Laborbereich, es ist allerdings nicht möglich, Einzelmessungen direkt am Bett des Patienten durchzuführen ohne den gesamten Analysator für andere Messungen zu blockieren.

Aufgabe der vorliegenden Erfindung ist es, ausgehend von den eingangs beschriebenen Analysatoren bzw. Analysensystemen ein flexibles, erweiterbares Analysensystem zur Analyse medizinischer Proben vorzuschlagen, welches die Vorteile gut handhabbarer, kompakter Einzelgeräte mit jenen eines Mehrkomponentenanalysators verbindet.

Diese Aufgabe wird erfindungsgemäß dadurch gelöst, dass ein oder mehrere, autonome Einzelanalysatoren zur Messung jeweils eines Probenparameters oder einer Parametergruppe und eine rechnergestützte Zentraleinheit mit einer Ein- und Ausgabeeinheit vorgesehen sind, dass die Einzelanalysatoren in einer ersten Position, der Ladeposition, mit der Zentraleinheit gekoppelt sind, sowie dass die Einzelanalysatoren aus der Ladeposition entnehmbar und in einer zweiten, vorzugsweise patientenseitigen Messposition einsetzbar sind. Erfindungsgemäß weist somit das Analysensystem handliche benutzerfreundliche Einzelanalysatoren sowie eine rechnergestützte Zentraleinheit auf, welche als Ladestation für die Einzelanalysatoren dient. Mit den Einzelanalysatoren in der Ladeposition kann das Analysensystem als flexibel erweiterbarer, automatischer Mehrkomponentenanalysator verendet werden, dessen Komponenten problemlos gegen andere ausgetauscht werden können. Mit einem Handgriff können die Einzelanalysatoren aus der Ladeposition entnommen und in eine vorzugsweise patientenseitige Messposition gebracht und somit dezentral zur Probenanalyse eingesetzt werden. Im Analysensystem können sowohl Einzelanalysatoren zur Messung unterschiedlicher Parameter bzw. Parametergruppen als auch mehrere gleichartige Einzelanalysatoren zur Messung derselben Parametergruppe verwendet werden, falls der Probendurchsatz erhöht werden soll. So ist es denkbar, z. B. mehrere Einzelanalysatoren zur Messung der Blutgase zu verwenden, wobei einige davon in patientenseitigen Messpositionen eingesetzt und andere in der Ladeposition an die Zentraleinheit gekoppelt sind und für deren Messeinsatz konditioniert werden.

Von besonderem Vorteil bei erfindungsgemäßen Analysensystem ist ein Bussystem, welches in der Ladeposition den lösbaren Kontakt der Einzelanalysatoren zur Zentraleinheit sowie den Kontakt zwischen den Einzelanalysatoren herstellt.

In einer einfachen Ausgestaltung der Erfindung weist das Bussystem beispielsweise einen Datenbus zur Herstellung einer Datenverbindung zwischen den Einzelkomponenten des Analysensystems auf. Weiters kann ein Energieversorgungsbus in das Bussystem integriert sein, so dass aufladbare Energiespeicher, beispielsweise Akkusysteme, in den Einzelanalysatoren in der Ladeposition aufgeladen werden können.

In weiterer Ausgestaltung der Erfindung ist es möglich, dass das Bussystem einen Fluidbus zum Austausch von Wasch-, Kalibrier- und Qualitätskontrollmitteln zwischen den Einzelkomponenten aufweist, so dass die einzelnen Medien je nach Bedarf aus Vorratsbehältern in der Zentraleinheit über das Bussystem zu den Einzelanalysatoren transportiert werden können.

Weiters ist es möglich, im Bussystem einen Probenbus zum Austausch der Probenflüssigkeit zwischen den Einzelkomponenten vorzusehen, so dass eine in der Zentraleinheit oder in einen der Einzelanalysatoren eingegebene Probe zur weiteren Analyse zu anderen Einzelanalysatoren transferiert werden kann.

Eine besonders vorteilhafte Ausführungsvariante sieht vor, dass das Bussystem Andockkonsolen für die Einzelanalysatoren aufweist, welche lösbare Steck- und Andockverbindungen für den Daten-, Energie-, Fluid- und/oder Probentransport aufweisen. Dabei können die Andockkonsolen untereinander (z.B. über die seitlichen Anschlussflächen), sowie mit der Zentraleinheit koppelbar sein, wobei lösbare Steck- und Andockverbindungen für den Daten-, Energie-, Fluid- und/oder Probentransport herstellbar sind. Das Analysensystem kann dadurch auf einfache Weise durch Ankoppeln weiterer Andockkonsolen erweitert werden. Es ist allerdings nicht notwendig, pro Einzelanalysator eine Andockkonsole vorzusehen, da bevorzugt alle Andockkonsolen und die Andockbereiche der Einzelanalysatoren baugleich ausgeführt sind, so dass für unterschiedliche Einzelanalysatoren nur eine Bauform von Andockkonsolen verwendbar ist. Von mehreren Einzelanalysatoren können somit einige in der Ladeposition an die Zentraleinheit gekoppelt sein, während andere, abseits von der Zentraleinheit, im autonomen Messbetrieb eingesetzt sind. Beim Entnehmen eines Einzelanalysators aus der Andockkonsole des Bussystems werden alle elektrischen Verbindungen sowie Fluidverbindungen gleichzeitig gelöst und die Fluidverbindungen im Bussystem sowie im Einzelanalysator durch Magnetventile, Dichtlippen oder elastische Dichtelemente selbsttätig verschlossen. Die Wiederherstellung aller Anschlüsse erfolgt durch einfaches Einsetzen des Einzelanalysators in eine Andockkonsole.

In einer weiteren erfindungsgemäßen Ausführungsvariante weist das Bussystem je Einzelanalysator eine von einem Steckplatz an der Zentraleinheit ausgehende zu einem Steckplatz am jeweiligen Analysator führende Kabel- bzw. Schlauchverbindung für den Daten-, Energie-, Fluid- und/oder Probentransport auf. Jeder Einzelanalysator muss in der Ladeposition lediglich durch eine Steckverbindung an die Zentraleinheit angeschlossen werden, wodurch alle im jeweiligen Bussystem integrierten Leitungen angeschlossen sind. Über den Datenbus des Bussystems erfolgt die Identifizierung der Einzelanalysatoren durch geeignete Softwareroutinen, welche in der Zentraleinheit gespeichert sind.

Erfindungsgemäß sind Einzelanalysatoren zur Messung der Parametergruppen Blutgase mit pH, Elektrolyte, Metabolite, CO-Oximetrie, Hämatologie, Gerinnung und Immunologie vorgesehen.

In einer weiteren Ausführungsvariante der Erfindung kann die Zentraleinheit ebenfalls eine Analyseneinrichtung zur Messung zumindest eines Probenparameters oder einer Parametergruppe der zu vermessenden medizinischen Probe aufweisen.

Um Einzelanalysatoren mit geringem Gewicht und kompakten Außenabmessungen realisieren zu können, kann die Zentraleinheit mit dem Bussystem in Verbindung stehende Vorratsbehälter für Wasch-, Kalibrier- und Qualitätskontrollmittel sowie einen Auffangbehälter für verbrauchte Proben sowie Wasch-, Kalibrier- und Qualitätskontrollmittel aufweisen.

Die autonomen Einzelanalysatoren weisen ein Eingabeelement (Folientastatur oder Barcodeleser, etc.) auf, um im Messbetrieb abseits von der Zentraleinheit Patientendaten eingeben zu können. Weiters ist ein Displayelement vorgesehen, um die Messergebnisse zumindest optisch anzuzeigen. Eine Weiterverarbeitung bzw. ein Ausdruck der Messergebnisse kann nach dem Einsetzen in die Zentraleinheit durch die in dieser Einheit vorgesehenen Auswerteeinrichtungen und Drucker erfolgen. Weiters können die Messdaten automatisch in die Patientendatei in der Zentraleinheit übertragen werden.

Weiters sind in der Zentraleinheit Kontroll- und Wartungseinrichtungen für die Einzelanalysatoren vorgesehen, welche über Softwareroutinen ausgehend von der Zentraleinheit ablaufen.

In einer besonders vorteilhaften Ausführungsvariante weist die Zentraleinheit einen Anschluss zur Datenfernübertragung, vorzugsweise einen Intranet- und/oder einen Internetanschluss auf. Dabei kann die Zentraleinheit eine Einrichtung zur automatisierten Erfassung der Betriebs- und/oder Verbrauchsmitteldaten, insbesondere zur Erfassung der Art und der maximalen Standzeit der verwendeten Betriebsmittel, sowie Art, Ablaufdaten und Mengenangaben der verwendeten Verbrauchsmittel eines Analysators, eine Einrichtung zur automatischen Berechnung der voraussichtlichen Analysenfrequenz aus den bisherigen Nutzungsdaten des Analysators oder eine Eingabeeinrichtung zur Eingabe der gewünschten Analysenfrequenz aufweisen, sowie weiters über eine Einrichtung zur Berechnung der pro Zeiteinheit in Abhängigkeit der Betriebs- und/oder Verbrauchsmitteldaten sowie der Analysenfrequenz benötigten Betriebs- und/oder Verbrauchsmittel verfügen, welche mit dem Anschluss zur Datenfernübertragung zur automatisierten Übertragung von Bestell-, Service- und Wartungsdaten in Verbindung steht.

Der besondere Vorteil dieser Ausführungsvariante liegt darin, dass wesentliche Teile des Betriebs- bzw. Verbrauchsmittelmanagements eines Analysensystems selbsttätig ablaufen. Die Erfassung der Daten nach dem Einsetzen neuer Sensorkassetten (Einheiten bzw. Module, welche durch den Anwender ausgetauscht werden können und meist mehrere Einzelsensoren für unterschiedliche Parameter in einer Messkammer enthalten) oder Verbrauchsmittel kann z.B. mit Hilfe eines Barcodelesers erfolgen oder durch ein Transpondersystem, bei welchem ein Speicherchip an oder in jeder Sensorkassette und jedem Behälter für Verbrauchsmittel angeordnet ist. Der Speicherchip, beispielsweise am Behälter für das Kalibriermittel, kann auch dafür verwendet werden, den jeweils aktuellen Füllstand des Kalibriermittels abzuspeichern. Weiters muss lediglich die gewünschte Analysenfrequenz, d. h. die pro Zeiteinheit geplanten Analysen, einmal eingegeben werden, bzw. kann auch vom Analysator aufgrund der bekannten Nutzungsdaten vorangegangener Nutzungsperioden vorgeschlagen und vom Benutzer quittiert werden. Es folgt eine automatische Berechnung der pro Zeiteinheit benötigten Betriebs- und Verbrauchsmittel und die Ermittlung eines optimalen Bestellzeitpunktes, wobei der Standort des Analysators und damit die zur Abwicklung der Bestellung und Lieferung benötigte Zeit berücksichtigt wird.

Die automatische Bestellung erfolgt beispielsweise über Internet, wobei ein direkter Kontakt zum Hersteller, zum Betriebsmittellieferanten bzw. zur Servicedienststelle oder zu einem Usercenter hergestellt wird. Das Verfahren ist für den Benutzer äußerst zeitsparend und sicher, da weder Bestellformulare ausgefüllt noch Kontaktadressen verwaltet werden müssen und zudem fehlerhafte Angaben bzw. Fehlbestellungen vermieden werden können.

Vorteilhafterweise kann der Internetzugang des Analysensystems in Zusammenhang mit den Ein- und Ausgabeelementen (Tastatur, Bildschirm, Drucker) der Zentraleinheit auch zur Bestellung für andere Produkte aus dem medizinischen oder klinischen Umfeld benutzt werden. Das Analysensystem kann somit eine Portalfunktion aufweisen und dem Benutzer auch das Management von Verbrauchsmaterialien für andere Geräte erleichtern bzw. dem Benutzer Zugang zu elektronischen Informationsmedien (Newsletters, Magazine usw.) anbieten.

Mit einer einmaligen Aktivierung im Setup-Programm des Analysensystems bzw. bei Vorliegen eines entsprechenden Wartungsvertrages können dem Benutzer aktiv genau jene Informationen und Updates mittels Internet zur Verfügung gestellt werden (Push-Technologie), die auf seine Bedürfnisse bzw. Systemkonfiguration abgestimmt sind und seine Arbeitsbedingungen optimieren.

Von Vorteil ist es weiters, dem Benutzer über die automatische Datenfernübertragung eine Helpfunktion anzubieten. Auf diese Art kann der Benutzer mit dem Hersteller, einem Usercenter, Usergroups, dem Betriebsmittellieferanten oder der Servicedienststelle in Verbindung treten (Chat-Room).

Weiters kann über den Internetanschluss eine Fernreparatur von Hard- oder Softwarekomponenten des Analysensystems bzw. der Zentraleinheit und der damit verbundenen Einzelanalysatoren durchgeführt werden. Der Fernreparatur geht eine Analyse allfälliger Fehlermeldungen bzw. die Analyse der letzten Kalibrations- und Qualitätskontrollzyklen voraus. Sodann können - ausgelöst von der über Internet in Verbindung stehenden Servicestelle - im Analysator vorkonfigurierte Serviceroutinen zur Behebung des Fehlers gestartet werden. Weiters können Programme überspielt werden, die unter Nutzung oder Sonderverwendung der im Analysensystem vorhandenen Baugruppen eine Behebung des Fehlers ermöglichen. Beispielsweise können durch wiederholtes Waschen der Probenwege, durch Umkehrung der Fließrichtung oder durch eine Änderung der Reihenfolge bestimmter Funktionsabläufe Ablagerungen oder Verunreinigungen im Probenweg entfernt werden, die durch routinemäßige Wasch- und Reinigungszyklen nicht erfasst werden.

Die automatisierte Bestellung der Betriebes- oder Verbrauchsmittel kann entweder nach einmaliger Aktivierung einer entsprechenden Funktion vollautomatisch von der Zentraleinheit abgewickelt oder durch das Analysensystem vorgeschlagen und vom Benutzer freigegeben werden.

Zur Berechnung der pro Zeiteinheit benötigten Betriebs- und Verbrauchsmittel kann unter anderem auch die gewünschte Reichweite (Vorratshaltung für einen gewünschten Zeitraum) und/oder die gewünschte Verfügbarkeit (geplante Auslastung) des Analysators eingegeben werden.

Aus den vom Analysensystem erhobenen bzw. berechneten Daten können Service- und Wartungsintervalle berechnet und über den Internetanschluss, entsprechende Service- und Wartungsdienste im Rahmen einer Fernwartung abgerufen bzw. bestellt werden. Soweit die Service- und Wartungsdienste Computersoftware betreffen, ist es in diesem Zusammenhang möglich, neue Versionen von Auswerteprogrammen bzw. Update-Versionen der Betriebssoftware automatisch abzurufen.

Mit Hilfe des Internetanschlusses der Zentraleinheit ist es weiters möglich, von Hardware- oder Software-Komponenten des Analysators ausgehende Fehlermeldungen zu erfassen, automatisch eine Fehlerdiagnose zu erstellen und über die automatische Datenfernübertragung entsprechende Service- und Wartungsdienste abzurufen bzw. zu bestellen.

Weiters ist vorgesehen, dass die Zentraleinheit eine Datenverbindung zu einem Laborinformationssystem (Laboratory Information System: LIS), zu einem Krankenhausinformationssystem (Hospital Information System: HIS), und/oder zu weiteren Laborgeräten (LS) ohne eigenen Internetanschluss aufweist.

In einer weiteren Ausführungsvariante ist vorgesehen, dass die Zentraleinheit und die Einzelanalysatoren Sende- und Empfangseinrichtungen für den drahtlosen Datentransfer aufweisen, so dass eine Datenverbindung auch dann aufrecht erhalten wird, wenn sich der Einzelanalysator in einer von der Zentraleinheit entfernten Messposition befindet. Der Datentransfer zwischen der Zentraleinheit und den Einzelanalysatoren sowie die Datenverbindung zu den Informationssystemen LIS bzw. HIS kann mittels Funk im 2,4 GHz Bereich erfolgen, wobei das lizenzfreie ISM-Band (Industrial-, Scientific-, Medical-Band) genutzt wird.

Die Erfindung wird im folgenden anhand von Zeichnungen näher erläutert. Es zeigen:
- Fig. 1: in schematischer Darstellung ein Analysensystem zur Analyse medizinischer Proben sowie die
- Fig. 2 und 3: Ausführungsvarianten des Analysensystems nach Fig. 1.

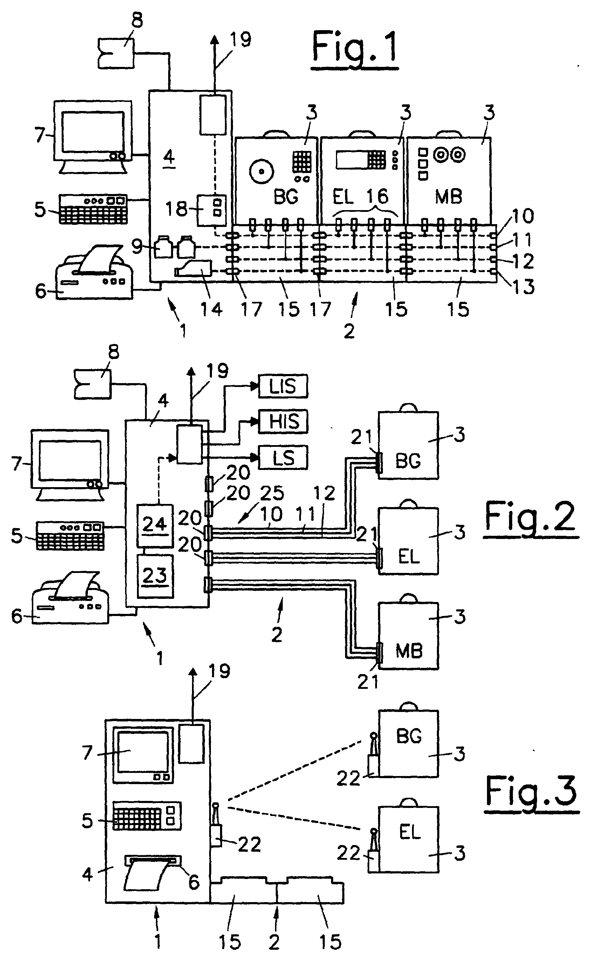

Das erfindungsgemäße Analysensystem zur Analyse von Körperflüssigkeiten gemäß Fig. 1 weist eine Zentraleinheit 1 sowie damit über ein Bussystem 2 verbundene autonome Einzelanalysatoren 3 auf. Die Einzelanalysatoren 3, beispielsweise ein Blutgasanalysator BG, ein Elektrolytanalysator EL sowie ein Analysator für unterschiedliche Metabolite MB, sind in einer ersten Position, der in Fig. 1 dargestellten Ladeposition, mit der Zentraleinheit 1 zu einem Mehrkomponentenanalysator gekoppelt. Aus dieser Ladeposition können die Einzelanalysatoren 3 mit Hilfe eines Griffelementes entnommen und in eine von der Zentraleinheit 1 entfernte Messposition transferiert werden.

Die Zentraleinheit 1 weist einen Computer 4 mit entsprechender Auswerte- und Steuersoftware, Datenbanken, etc. auf sowie integrierte bzw. an den Computer angeschlossene Ein- und Ausgabeelemente, wie Tastatur 5, Drucker 6 und Bildschirm 7. Weiters kann die Zentraleinheit 1 über einen Barcodeleser 8 sowie über eine elektronische Verbindung bzw. einen Anschluss 19 zur Datenfernübertragung (Anschluss an den Zentralrechner des Labors bzw. der Klinik, Intranet, Internet, etc.) verfügen. Die Einzelanalysatoren 3 weisen Probeneingabeeinrichtungen sowie einfache Dateneingabe- bzw. Displayelemente auf, sodass deren autonome Verwendung, ohne Verbindung zur Zentraleinheit 1 möglich ist. Falls notwendig, können in den Einzelanalysatoren 3 hier nicht dargestellte Zwischenspeicher für die Wasch- und Kalibriermedien vorgesehen sein.

Das Bussystem 2 weist zumindest einen Datenbus 10 zur Verbindung der Zentraleinheit 1 mit den autonomen Einzelanalysatoren 3 auf. Weiters ist im Ausführungsbeispiel ein Fluidbus 11 zum Austausch von Wasch-, Kalibrier- und Qualitätskontrollmitteln zwischen den Einzelanalysatoren 3 vorgesehen, wobei auch ein Anschluss zur Zentraleinheit 1 vorgesehen sein kann.

Falls die Einzelanalysatoren 3 über keine separate Energieversorgung verfügen, kann auch ein von der Zentraleinheit 1 ausgehender Energieversorgungsbus 12 im Bussystem 2 integriert sein. Schließlich ist ein Probenbus 13 vorgesehen, welcher zum Austausch der Probe zwischen den Einzelanalysatoren dient und einen Anschluss zu einem Auffang- bzw. Abfallbehälter 14 in der Zentraleinheit 1 aufweist.

In der Ausführungsvariante gemäß Fig. 1 besteht das Bussystem 2 im wesentlichem aus baugleich ausgeführten Andockkonsolen 15 für jeden Einzelanalysator 3, welche lösbare Steck- und Andockverbindungen 16 für die einzelnen Buskomponenten aufweisen. In einzelnen oder jeder der Andockkonsolen 15 können mit dem Fluidbus 11 verbundene Behälter für ein Wasch-, Kalibrier- und Qualitätskontrollmittel vorgesehen sein. Derartige Behälter 9 können - wie in Fig. 1 dargestellt - auch zur Versorgung aller Einzelanalysatoren in der Zentraleinheit 1 untergebracht sein. Jeder Einzelanalysator 3 kann somit auf einfache Weise aus seiner Andockkonsole 15 entnommen und an einem anderen Standort als autonomer Analysator für bestimmte Parameter oder Parametergruppen eingesetzt werden. Um den Probendurchsatz zu erhöhen ist es auch möglich, in einem Analysensystem mehrere gleichartige Einzelanalysatoren 3 einzusetzen.

Über die in der Zentraleinheit 1 angeordnete Kontroll- und Wartungseinrichtung 18 können bestimmte Wartungs- und Servicefunktionen für die Einzelanalysatoren 3 ausgeführt werden, weiters ist es möglich über den Datenbus eine automatische Konfiguration des Gesamtsystems vorzunehmen. Die Kontroll- und Wartungseinheit 18 ist mit einem Anschluss 19 zur Datenfernübertragung ausgestattet, so dass beispielsweise über einen Internetanschluss ein Remote-Access für Ferndiagnose und Fernwartung hergestellt werden kann.

Da die Andockkonsolen 15 über seitliche Anschlussflächen untereinander sowie mit der Zentraleinheit 1 modulartig koppelbar sind, wobei ebenfalls lösbare Steck- und Andockverbindungen 17 für die einzelnen Buskomponenten hergestellt werden, kann das Analysensystem jederzeit um zusätzliche Andockkonsolen 15 und die jeweils benötigten Einzelanalysatoren 3 erweitert werden.

Die Ausführungsvariante gemäß Fig. 2 unterscheidet sich von jener nach Fig. 1 dadurch, dass das Bussystem 2 die Einzelanalysatoren 3 sternförmig an die Zentraleinheit 1 anbindet. Der Datenbus 10, der Fluidbus 11 und der Energieversorgungsbus 12 sind jeweils in Kabel- bzw. Schlauchverbindungen 25 zusammengefasst, welche von Steckplätzen 20 an der Zentraleinheit 1 zu Steckplätzen 21 an den Einzelanalysatoren 3 führen. Nach dem Abstecken des Einzelanalysators 3 kann dieser als autonomer Analysator eingesetzt werden. Das System kann durch den Anschluss zusätzlicher Einzelanalysatoren an die Steckplätze 20 der Zentraleinheit 1 erweitert werden.

Wie anhand der Ausführungsvariante gemäß Fig. 2 dargestellt, kann die Zentraleinheit 1 eine Einrichtung 23 zur Berechnung der voraussichtlichen Analysenfrequenz aus den bisherigen Nutzungsdaten sowie eine Einrichtung 24 zur Berechnung der pro Zeiteinheit benötigten Betriebs- und/oder Verbrauchsmittel aufweisen. Das Analysensystem nützt den Anschluss 19 zur Datenfernübertragung zur automatisierten Übertragung von den in der Einrichtung 24 erhobenen Bestell-, Service- und Wartungsdaten. Es ist auch möglich, die gewünschte oder voraussichtliche Analysenfrequenz über die Eingabeeinrichtung bzw. Tastatur 5 einzugeben. Der Analysator kann weiters eine Datenverbindung zu einem Laborinformationssystem LIS, zu einem Krankenhausinformationssystem HIS und zu weiteren Laborgeräten LS aufweisen. Insbesondere können Geräte angebunden sein, welche über keinen eigenen Internetanschluss verfügen.

Der Datentransfer zwischen der Zentraleinheit 1 und den Einzelanalysatoren 3 sowie die Anknüpfung zu einem Laborinformationssystem LIS bzw. Krankenhausinformationssystem HIS kann auch drahtlos mittels Funk im 2,4 GHz-Bereich unter Nutzung des ISM-Bandes erfolgen.

Eine weitere, kompakte Ausführungsvariante der Erfindung ist in Fig. 3 dargestellt, bei welcher die Einzelanalysatoren 3 in einer von der Zentraleinheit 1 entfernten Messposition dargestellt sind. Sende- und Empfangseinrichtungen 22 an der Zentraleinheit 1 und den Einzelanalysatoren 3 ermöglichen in der Messposition einen drahtlosen Datentransfer zwischen den Einzelkomponenten.

## Patentansprüche

1. Analysensystem zur Analyse medizinischer Proben, vorzugsweise zur Analyse von Körperflüssigkeiten **dadurch gekennzeichnet, dass** ein oder mehrere, autonome Einzelanalysatoren (3) zur Messung jeweils eines Probenparameters oder einer Parametergruppe und eine rechnergestützte Zentraleinheit (1) mit einer Ein- und Ausgabeeinheit (5, 6, 7, 8) vorgesehen sind, dass die Einzelanalysatoren (3) in einer ersten Position, der Ladeposition, mit der Zentraleinheit (1) gekoppelt sind, sowie dass die Einzelanalysatoren (3) aus der Ladeposition entnehmbar und in einer zweiten, vorzugsweise patientenseitigen Messposition einsetzbar sind.

2. Analysensystem nach Anspruch 1, **dadurch gekennzeichnet, dass** ein Bussystem (2) vorgesehen ist, welches in der Ladeposition den lösbaren Kontakt zwischen den Einzelanalysatoren (3) sowie den Kontakt der Einzelanalysatoren (3) zur Zentraleinheit (1) herstellt.

3. Analysensystem nach Anspruch 2, **dadurch gekennzeichnet, dass** das Bussystem (2) einen Datenbus (10) zur Herstellung einer Datenverbindung zwischen den Einzelkomponenten (1, 3) aufweist

4. Analysensystem nach Anspruch 2 oder 3, **dadurch gekennzeichnet, dass** das Bussystem (2) einen Fluidbus (11) zum Austausch von Wasch-, Kalibrier- und Qualitätskontrollmitteln zwischen den Einzelkomponenten (1, 3) aufweist.

5. Analysensystem nach einem der Ansprüche 2 bis 4, **dadurch gekennzeichnet, dass** das Bussystem (2) einen Energieversorgungsbus (12) aufweist.

6. Analysensystem nach einem der Ansprüche 2 bis 5, **dadurch gekennzeichnet, dass** das Bussystem (2) einen Probenbus (13) zum Austausch der Probenflüssigkeit zwischen den Einzelkomponenten (1, 3) aufweist.

7. Analysensystem nach einem der Ansprüche 2 bis 6, **dadurch gekennzeichnet, dass** das Bussystem (2) Andockkonsolen (15) für die Einzelanalysatoren (3) aufweist, welche lösbare Steck- und Andockverbindungen (16) für den Daten-, Energie-, Fluid- und/oder Probentransport aufweisen.

8. Analysensystem nach Anspruch 7, **dadurch gekennzeichnet, dass** die Andockkonsolen (15) untereinander, sowie mit der Zentraleinheit (1) koppelbar sind, wobei lösbare Steck- und Andockverbindungen (17) für den Daten-, Energie-, Fluid- und/oder Probentransport herstellbar sind.

9. Analysensystem nach Anspruch 7 oder 8, **dadurch gekennzeichnet, dass** alle Andockkonsolen (15) und die Andockbereiche der Einzelanalysatoren (3) baugleich ausgeführt sind.

10. Analysensystem nach einem der Ansprüche 2 bis 6, **dadurch gekennzeichnet, dass** das Bussystem (2) je Einzelanalysator (3) eine von einem Steckplatz (20) an der Zentraleinheit (1) ausgehende, zu einem Steckplatz (21) am jeweiligen Einzelanalysator (3) führende Kabel-bzw. Schlauchverbindung (23) für den Daten-, Energie-, Fluid- und/oder Probentransport aufweist.

11. Analysensystem nach einem der Ansprüche 1 bis 10, **dadurch gekennzeichnet, dass** Einzelanalysatoren (3) zur Messung der Parametergruppen Blutgase mit pH, Elektrolyte, Metabolite, CO-Oximetrie, Hämatologie, Gerinnung und Immunologie vorgesehen sind.

12. Analysensystem nach einem der Ansprüche 1 bis 11, **dadurch gekennzeichnet, dass** die Zentraleinheit (1) eine Analyseneinrichtung (3') zur Messung zumindest eines Probenparameters oder einer Parametergruppe der medizinischen Probe aufweist.

13. Analysensystem nach einem der Ansprüche 1 bis 12, **dadurch gekennzeichnet, dass** die Zentraleinheit (1) eine Kontroll- und Wartungseinrichtung (18) für die Einzelanalysatoren (3) aufweist.

14. Analysensystem nach einem der Ansprüche 1 bis 13, **dadurch gekennzeichnet, dass** die Zentraleinheit (1) Vorratsbehälter (9) für Wasch-, Kalibrier- und Qualitätskontrollmittel sowie einen Auffangbehälter (14) für verbrauchte Proben, und Wasch-, Kalibrier- und Qualitätskontrollmittel aufweist.

15. Analysensystem nach einem der Ansprüche 1 bis 14, **dadurch gekennzeichnet, dass** die Zentraleinheit (1) einen Anschluss (19) zur Datenfernübertragung, vorzugsweise einen Intranet- und/oder einen Internetanschluss aufweist.

16. Analysensystem nach Anspruch 15, **dadurch gekennzeichnet, dass** die Zentraleinheit (1) eine Einrichtung (8) zur automatisierten Erfassung der Betriebs- und/oder Verbrauchsmitteldaten, insbesondere zur Erfassung der Art und der maximalen Standzeit der verwendeten Betriebsmittel, sowie Art, Ablaufdaten und Mengenangaben der verwendeten Verbrauchsmittel, eine Einrichtung (23) zur automatischen Berechnung der voraussichtlichen Analysenfrequenz aus den bisherigen Nutzungsdaten des Analysators oder eine Eingabeeinrichtung (5) zur Eingabe der gewünschten Analysenfrequenz aufweist, sowie dass weiters eine Einrichtung (24) zur Berechnung der pro Zeiteinheit in Abhängigkeit der Betriebs- und/oder Verbrauchsmitteldaten sowie der Analysenfrequenz benötigten Betriebs- und/oder Verbrauchsmittel vorgesehen ist, welche mit dem Anschluss zur Datenfernübertragung (19) zur automatisierten Übertragung von Bestell-, Service- und Wartungsdaten in Verbindung steht.

17. Analysensystem nach einem der Ansprüche 1 bis 16, **dadurch gekennzeichnet, dass** die Zentraleinheit (1) eine Datenverbindung zu einem Laborinformationssystem (LIS), zu einem Krankenhausinformationssystem (HIS), und/oder zu weiteren Laborgeräten (LS) aufweist.

18. Analysensystem nach einem der Ansprüche 1 bis 17, **dadurch gekennzeichnet, dass** die Zentraleinheit (1) und die Einzelanalysatoren (3) Sende- und Empfangseinrichtungen (22) für den drahtlosen Datentransfer aufweisen.

19. Analysensystem nach Anspruch 17 oder 18, **dadurch gekennzeichnet, dass** der Datentransfer mittels Funk im 2,4 GHz Bereich erfolgt und das ISM-Band (Industrial-, Scientific-, Medical-Band) nutzt.
